# EUROPEAN PATENT APPLICATION

(11) **EP 4 786 460 A1**
(43) Date of publication of application: **05.08.2026**
(21) Application number: 24870949.5
(22) Date of filing: 27.09.2024
(51) Int. Cl.: C07D 401/04

(54) **PREPARATION METHOD FOR ANTI-TUMOR DRUG INTERMEDIATE**

(30) Priority: 28.09.2023 CN 202311265672
(71) Applicant: Porton Pharma Solutions Ltd., Chongqing 401220 (CN)
(72) Inventor: XIA, Yingqi, Chongqing 401220 (CN); HUANG, Qiang, Chongqing 401220 (CN); WANG, Zhenglin, Chongqing 401220 (CN); TANG, Yuanfu, Chongqing 401220 (CN); ZENG, Min, Chongqing 401220 (CN); ZHAO, Shibing, Chongqing 401220 (CN)
(74) Representative: Scholl, Matthias
(86) International application number: PCT/CN2024/121707
(87) International publication number: WO 2025/067412

(57) **Abstract**

A preparation method for an anti-tumor drug intermediate, comprising the following step: when a monovalent copper salt is used as a catalyst and a pyrrolidinol compound is used as a ligand, a compound of formula II reacts with a compound of formula III, i.e., 4-morpholinopiperidine, to prepare a compound of formula I or a salt thereof. Compared with the prior art, the present application uses a monovalent copper catalyst and uses a pyrrolidinol compound as a ligand, thereby reducing the cost of a catalytic system, and increasing the yield. In addition, the reaction achieves a high purity and fewer impurities.

## Description

### DESCRIPTION

The disclosure relates to the field of medicine, and more particularly to a method for preparing a compound of formula I, which is an intermediate of alectinib, or a salt thereof.

Alectinib is an oral anaplastic lymphoma kinase (ALK) inhibitor developed by Genentech, a member of the Roche Group, for the treatment of patients with ALK-positive locally advanced or metastatic non-small cell lung cancer.

Compound of formula I is a key intermediate in the synthetic route disclosed by the originator company (US9126931B2). The intermediate is prepared from an aryl iodide (II) and 4-morpholinopiperidine (III) under palladium-catalyzed carbon-nitrogen bond coupling reaction conditions.

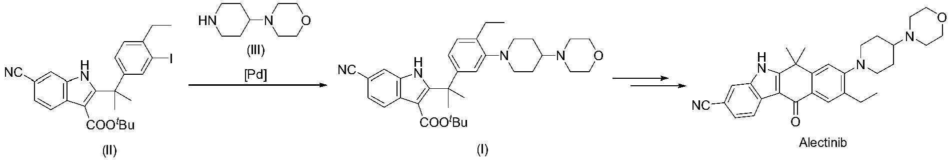

The loading of the metal palladium catalyst in the above reaction is relatively high, ranging from 1.5% to 2.0%. Moreover, metallic palladium is expensive, which leads to high preparation costs for the compound (I) and consequently increases the production cost of alectinib. Furthermore, palladium catalytic systems have stringent requirements for anhydrous and oxygen-free conditions, making industrial production operations relatively difficult. In view of the drawbacks of the existing process, there is a need for a catalytic system with low cost and industrial applicability for preparing the key intermediate (I) of alectinib.

Chinese Patent Publication No. CN115677659A discloses a method for preparing intermediate (I) from intermediate (II) using a copper catalyst. However, the products prepared with the copper catalyst and ligand suffer from low yield and long reaction time, and there remains a need for a more efficient copper catalytic system.

To solve the technical problems in the related art, the disclosure provides a method for preparing an anti-tumor drug intermediate. This method uses a cuprous salt as a catalyst and a pyrrole alcohol compound as a ligand, thereby reducing the cost of expensive heavy metal catalysts, while improving the yield of the intermediate. Moreover, the method is simple, environmentally friendly, and amenable to industrial-scale production.

The following technical solution are adopted in the disclosure.

A method for preparing an intermediate of an anti-cancer drug, comprises:

reacting a compound of formula II with a compound of formula III, 4-morpholinopiperidine, in the presence of a cuprous salt as a catalyst and a pyrrole alcohol compound as a ligand, to obtain a compound of formula I or a salt thereof:

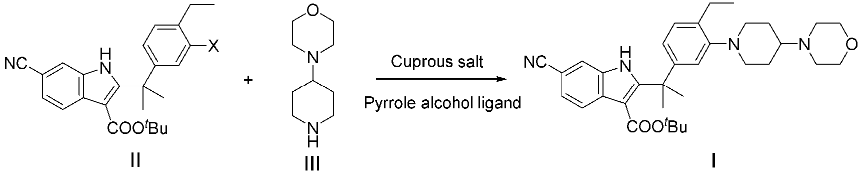

In a class of this embodiment, the pyrrole alcohol compound has a formula as follows:

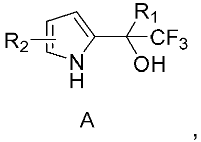

R₁ is selected from the group consisting of H, methyl, ethyl, straight-chain or branched C₃-C₅ alkyl, benzyl, phenyl, naphthyl, and substituted phenyl; wherein the substituted phenyl is substituted at an ortho, meta, or para position with a substituent selected from the group consisting of methyl, methoxy, and isopropyl; and

R₂ is selected from the group consisting of H, methyl, ethyl, straight-chain or branched C₃-C₅ alkyl, phenyl, benzyl, methoxy, and ethoxy, and R₂ is located at any substitutable position on a pyrrole ring.

In a class of this embodiment, R₁ is selected from the group consisting of H, methyl, ethyl, straight-chain or branched C₃-C₅ alkyl, and phenyl; R₂ is selected from the group consisting of H, methyl, ethyl, and phenyl, and R₂ is located at any position selected from the 3-position, 4-position, or 5-position of the pyrrole ring.

In a class of this embodiment, the pyrrole alcohol compound comprises the following formulas:

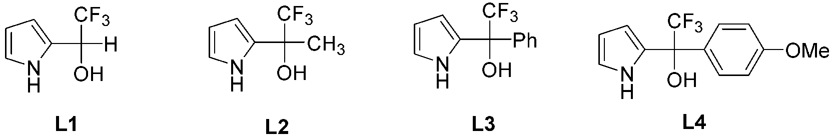

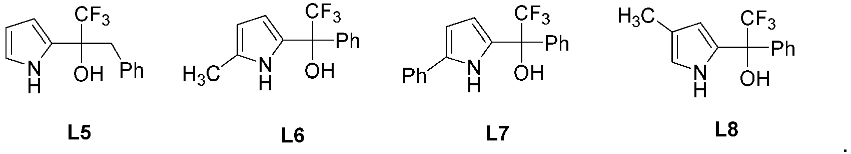

In a class of this embodiment, the cuprous salt is selected from the group consisting of cuprous iodide, cuprous bromide, cuprous chloride, cuprous oxide, and copper thiophene-2-carboxylate.

In a class of this embodiment, the molar ratio of the cuprous salt to the compound of formula II is from 1: 1 to 1: 20.

In a class of this embodiment, the molar ratio of the cuprous salt to the compound of formula II is from 1: 1 to 1: 15.

In a class of this embodiment, reacting the compound of formula II with the compound of formula III is carried out at a temperature ranging from 80°C to 130°C for a reaction time ranging from 1 hour to 15 hours.

In a class of this embodiment, reacting the compound of formula II with the compound of formula III is carried out at a temperature ranging from 90°C to 125°C for a reaction time ranging from 1 hour to 12 hours.

In a class of this embodiment, reacting the compound of formula II with the compound of formula III further involves a base selected from the group consisting of triethylamine, diisopropylethylamine, 1,8-diazabicyclo[5.4.0]undec-7-ene, potassium phosphate, potassium carbonate, cesium carbonate, and potassium hydroxide, and a molar ratio of the base to the compound of formula II is from 1: 1 to 6: 1.

Compared to the prior art, the method of the disclosure employs a monovalent copper catalyst and a pyrrole alcohol compound as a ligand, which reduces the cost of the catalytic system and improves the yield. Moreover, the reaction affords high purity and fewer impurities.
FIG. 1 shows a ¹H NMR spectrum of a hydrochloride salt of the compound of formula I;
FIG. 2 shows a ¹³C NMR spectrum of a hydrochloride salt of the compound of formula I; and
FIG. 3 shows an HPLC spectrum of a product prepared in Example 1.

The disclosure provides a method for preparing an intermediate of an anti-cancer drug, the method comprising:
reacting a compound of formula II with a compound of formula III, 4-morpholinopiperidine, in the presence of a cuprous salt as a catalyst and a pyrrole alcohol compound as a ligand, to obtain a compound of formula I or a salt thereof:

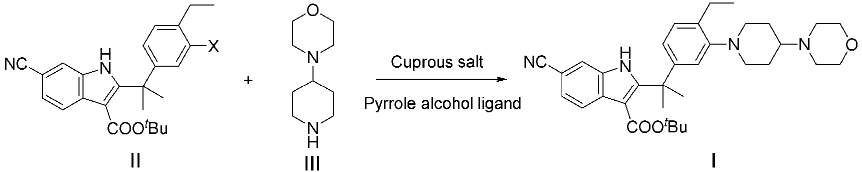

Furthermore, the pyrrole alcohol compound has a formula as follows: 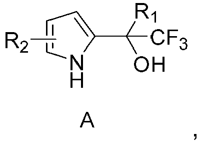
R₁ is selected from the group consisting of H, methyl, ethyl, straight-chain or branched C₃-C₅ alkyl, benzyl, phenyl, naphthyl, and substituted phenyl; wherein the substituted phenyl is substituted at an ortho, meta, or para position with a substituent selected from the group consisting of methyl, methoxy, and isopropyl; and
R₂ is selected from the group consisting of H, methyl, ethyl, straight-chain or branched C₃-C₅ alkyl, phenyl, benzyl, methoxy, and ethoxy, and R₂ is located at any substitutable position on a pyrrole ring.

Preferably, R₁ is selected from the group consisting of H, methyl, ethyl, straight-chain or branched C₃-C₅ alkyl, and phenyl; R₂ is selected from the group consisting of H, methyl, ethyl, and phenyl, and R₂ is located at any position selected from the 3-position, 4-position, or 5-position of the pyrrole ring.

Preferably, the pyrrole alcohol compound comprises the following formulas:

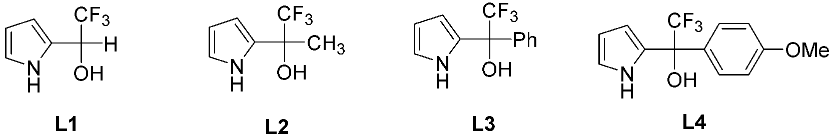

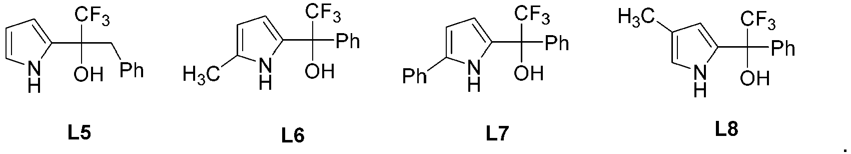

Furthermore, the cuprous salt is selected from the group consisting of cuprous iodide, cuprous bromide, cuprous chloride, cuprous oxide, and copper thiophene-2-carboxylate.

Furthermore, the molar ratio of the cuprous salt to the compound of formula II is from 1: 1 to 1: 20. In preferred embodiments, the molar ratio of the cuprous salt to the compound of formula II is from 1: 1 to 1: 15. In more preferred embodiments, the molar ratio of the cuprous salt to the compound of formula II is from 1: 2 to 1: 14. Further preferred embodiments include molar ratios selected from the group consisting of 1: 2, 1: 3, 1: 4, 1: 5, 1: 6, 1: 7, 1: 8, 1: 9, 1: 10, 1: 11, 1: 12, 1: 13, and 1: 14. If the amounts of the cuprous salt and the pyrrole alcohol ligand are too low, the reaction of the compound of formula II may be incomplete.

Optionally, the molar ratio of the monovalent copper salt to the pyrrole alcohol ligand is from 1: 1 to 1: 5, preferably from 1: 1 to 1: 3, and more preferably selected from the group consisting of 1: 1, 1: 2, and 1: 3.

In addition, if the reaction temperature is too low, the reaction of the compound of formula II may be incomplete, resulting in a decreased yield. Conversely, if the temperature is too high, the pyrrole alcohol ligand may decompose, also leading to incomplete reaction of the compound of formula II and an increase in by-products. The reaction is typically carried out at a temperature ranging from 80°C to 130°C, preferably from 90°C to 125°C. In more preferred embodiments, the reaction temperature is selected from the group consisting of 95°C, 100°C, 105°C, 110°C, 115°C, 120°C, and 125°C.

Optionally, the reaction is carried out for a period ranging from 1 hour to 15 hours. Preferably, the reaction time ranges from 1 hour to 12 hours. In more preferred embodiments, the reaction time is selected from the group consisting of 1 hour, 2 hours, 3 hours, 4 hours, 5 hours, 6 hours, 7 hours, 8 hours, 9 hours, 10 hours, 11 hours, and 12 hours.

Furthermore, the reaction conditions may optionally include a base. Suitable bases include, but are not limited to, triethylamine, diisopropylethylamine, 1,8-diazabicyclo[5.4.0]undec-7-ene, potassium phosphate, potassium carbonate, cesium carbonate, and potassium hydroxide. The molar ratio of the base to the compound of formula II is typically from 1: 1 to 6: 1. Preferred bases are triethylamine, potassium phosphate, potassium carbonate, cesium carbonate, and potassium hydroxide. A preferred molar ratio of the base to the compound of formula II is from 2: 1 to 5: 1, with more preferred ratios being 2: 1, 3: 1, 4: 1, and 5: 1. If the amount of inorganic base is too low, the reaction of the compound of formula II may be incomplete; if the amount of inorganic base is too high, an increase in impurities may occur.

Furthermore, suitable organic solvents for use in the reaction are polar aprotic solvents, including but not limited to acetonitrile, N,N-dimethylformamide, N,N-dimethylacetamide, N,N-dimethylpropyleneurea, 1,3-dimethyl-2-imidazolidinone, dimethyl sulfoxide, and sulfolane. Preferred solvents are N,N-dimethylacetamide, dimethyl sulfoxide, and sulfolane. These solvents are readily available and inexpensive.

### Example 1

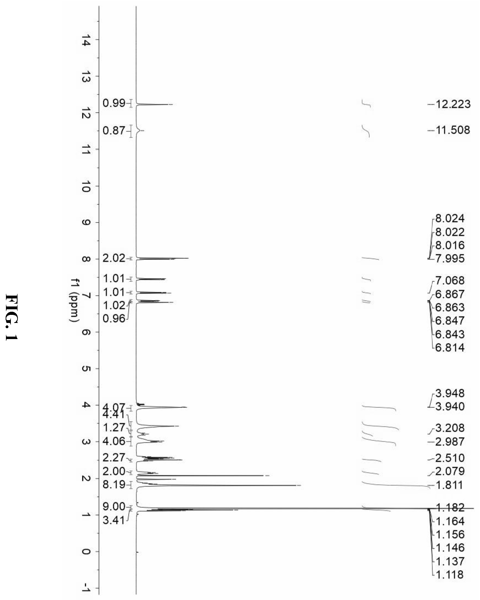

Copper(I) iodide (1.14 g, 6.0 mmol), pyrrole alcohol ligand 2,2,2-trifluoro-1-(1H-pyrrol-2-yl)ethan-1-ol (L1) (0.99 g, 6.0 mmol), tert-butyl 6-cyano-2-(2-(4-ethyl-3-iodophenyl)propan-2-yl)-1H-indole-3-carboxylate (10.29 g, 20.0 mmol), 4-morpholinopiperidine (4.26 g, 25.0 mmol), and potassium carbonate (11.06 g, 80.0 mmol) were placed in a dry reaction flask under a nitrogen atmosphere. N,N-Dimethylacetamide (120 mL) was added to the flask, and the reaction mixture was then heated to 120°C and stirred at this temperature for 12 hours. The reaction mixture was cooled to room temperature, and isopropyl acetate (480 mL) and a 15% aqueous ammonium chloride solution (120 mL) were added. The resulting mixture was stirred at room temperature for one hour. The aqueous layer was separated, and a saturated aqueous sodium chloride solution (120 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (200 mL), and the internal temperature of the solution was raised to 35°C. A mixture of pyridine hydrochloride (2.54 g, 22.0 mmol) in ethanol (3.0 mL) and acetone (6.0 mL) was added dropwise over 30 minutes to crystallize the desired product. The resulting slurry was stirred at 0°C for 3 hours. The wet powder was collected by filtration through a Büchner funnel and washed with acetone (50 mL). The wet powder was dried under reduced pressure at 40°C for 8 hours to obtain the hydrochloride salt of the compound of formula I (10.3 g, yield 86.8%, HPLC purity 99.1%). See FIG. 1.

¹H NMR (400MHz, DMSO): δ 12.22 (s, 1H), 11.50 (s, 1H), 8.02-7.99 (m, 2H), 7.45 (dd, *J*₁ = 8.4 Hz, *J*₂ = 1.2 Hz, 1H), 7.07 (d, *J* = 8.0 Hz, 1H), 6.85 (dd, *J*₁ = 8.0 Hz, *J*₂ = 1.6 Hz, 1H), 6.81 (s, 1H), 3.94 (d, *J* = 3.2 Hz, 4H), 3.42 (s, 4H), 3.20 (t, *J =* 11.6 Hz, 1H), 3.01-2.98 (m, 4H), 2.51-2.48 (m, 2H), 2.15 (d, *J =* 10.80 Hz, 2H), 1.88-1.81 (m, 8H), 1.18 (s, 9H), 1.13 (t, *J* = 7.60 Hz, 3H) ppm; as shown in FIG. 1.

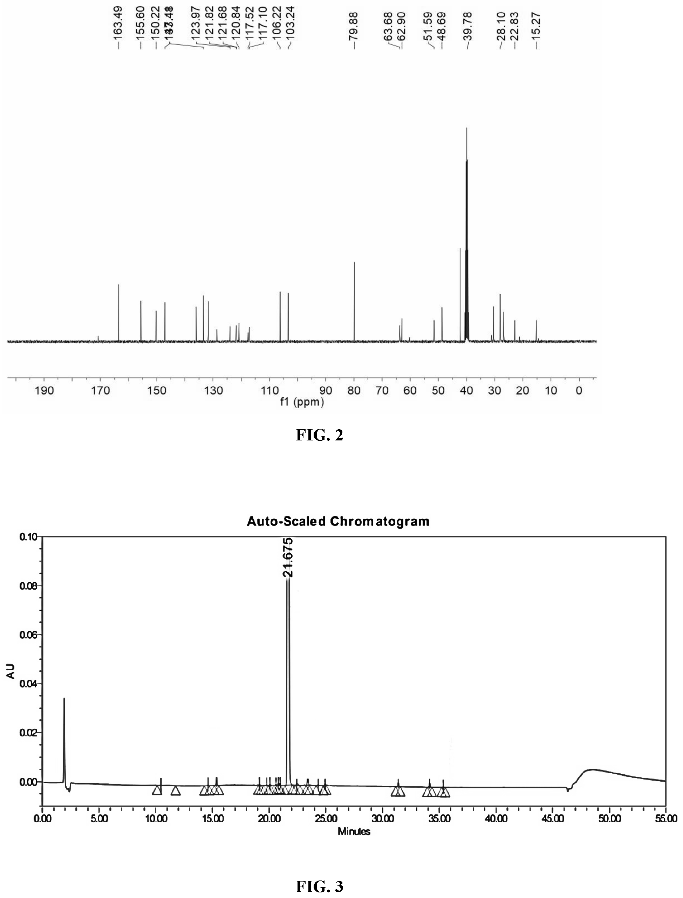

¹³C NMR (100MHz, DMSO): δ 163.4, 155.6, 150.2, 147.1, 136.0, 133.4, 131.7, 128.6, 123.9, 121.8, 121.6, 120.8, 117.5, 117.1, 106.2, 103.2, 79.8, 63.6, 62.9, 51.5, 48.6, 42.2, 30.4, 28.1, 26.8, 22.8, 15.2 ppm; as shown in FIG. 2.

ESI HRMS: calcd for C₃₄H₄₄N₄O₃ + H: 557.3492; found: 557.3496.

### Example 2

Copper(I) iodide (0.76 g, 4.0 mmol), pyrrole alcohol ligand 1,1,1-trifluoro-2-(1H-pyrrol-2-yl)propan-2-ol (L2) (0.72 g, 4.0 mmol), tert-butyl 6-cyano-2-(2-(4-ethyl-3-iodophenyl)propan-2-yl)-1H-indole-3-carboxylate (10.29 g, 20.0 mmol), 4-morpholinopiperidine (4.09 g, 24.0 mmol), and potassium phosphate (16.98 g, 80.0 mmol) were placed in a dry reaction flask under a nitrogen atmosphere. Sulfolane (100 mL) was added to the flask, and the reaction mixture was then heated to 110°C and stirred at this temperature for 8 hours. The reaction mixture was cooled to room temperature, and isopropyl acetate (400 mL) and a 15% aqueous ammonium chloride solution (60 mL) were added. The resulting mixture was stirred at room temperature for 1 hour. The aqueous layer was separated, and a saturated aqueous sodium chloride solution (100 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (180 mL), and the internal temperature of the solution was raised to 35°C. A mixture of pyridine hydrochloride (2.54 g, 22.0 mmol) in ethanol (3.0 mL) and acetone (6.0 mL) was added dropwise over 30 minutes to crystallize the desired product. The resulting slurry was stirred at 0°C for 3 hours. The wet powder was collected by filtration through a Büchner funnel and washed with acetone (40 mL). The wet powder was dried under reduced pressure at 40°C for 6 hours to obtain the hydrochloride salt of the compound of formula I (11.6 g, yield 97.8%, HPLC purity 98.7%).

### Example 3

Copper(I) iodide (0.57 g, 3.0 mmol), pyrrole alcohol ligand 2,2,2-trifluoro-1-phenyl-1-(1H-pyrrol-2-yl)ethan-1-ol (L3) (0.73 g, 3.0 mmol), tert-butyl 6-cyano-2-(2-(4-ethyl-3-iodophenyl)propan-2-yl)-1H-indole-3-carboxylate (6.17 g, 12.0 mmol), 4-morpholinopiperidine (3.07 g, 18.0 mmol), and potassium phosphate (10.19 g, 48.0 mmol) were placed in a dry reaction flask under a nitrogen atmosphere. Sulfolane (60.0 mL) was added to the flask, and the reaction mixture was then heated to 105°C and stirred at this temperature for 5 hours. The reaction mixture was cooled to room temperature, and isopropyl acetate (300 mL) and a 15% aqueous ammonium chloride solution (40 mL) were added. The resulting mixture was stirred at room temperature for 1 hour. The aqueous layer was separated, and a saturated aqueous sodium chloride solution (40 mL) was added to the organic layer. The mixture was stirred at room temperature for 1 hour. The aqueous layer was separated, and the obtained organic layer was concentrated under reduced pressure at an external temperature of 50°C. The residue was dissolved in acetone (90 mL), and the internal temperature of the solution was raised to 35°C. A mixture of pyridine hydrochloride (1.53 g, 13.2 mmol) in ethanol (1.5 mL) and acetone (3.0 mL) was added dropwise over 30 minutes to crystallize the desired product. The resulting slurry was stirred at 0°C for 3 hours. The wet powder was collected by filtration through a Büchner funnel and washed with acetone (30 mL). The wet powder was dried under reduced pressure at 50°C for 8 hours to obtain the hydrochloride salt of the compound of formula I (6.73 g, yield 94.5%, HPLC purity 99.0%).

### Example 4

Copper(I) iodide (0.38 g, 2.0 mmol), 1,1,1-trifluoro-3-phenyl-2-(1H-pyrrol-2-yl)propan-2-ol (L5) (0.51 g, 2.0 mmol), tert-butyl 6-cyano-2-(2-(4-ethyl-3-iodophenyl)propan-2-yl)-1H-indole-3-carboxylate (10.29 g, 20.0 mmol), 4-morpholinopiperidine (3.75 g, 22.0 mmol), and cesium carbonate (26.07 g, 80.0 mmol) were placed in a dry reaction flask under a nitrogen atmosphere. Dimethyl sulfoxide (80 mL) was added to the flask, and the reaction mixture was then heated to 110°C and stirred at this temperature for 5 hours. The reaction mixture was cooled to room temperature, and isopropyl acetate (320 mL) and a 15% aqueous ammonium chloride solution (40 mL) were added. The resulting mixture was stirred at room temperature for 1 hour. The aqueous layer was separated, and a saturated aqueous sodium chloride solution (80 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (180 mL), and the internal temperature of the solution was raised to 35°C. A mixture of pyridine hydrochloride (2.54 g, 22.0 mmol) in ethanol (3.0 mL) and acetone (6.0 mL) was added dropwise over 30 minutes to crystallize the desired product. The resulting slurry was stirred at 0°C for 3 hours. The wet powder was collected by filtration through a Büchner funnel and washed with acetone (40 mL). The wet powder was dried under reduced pressure at 40°C for 5 hours to obtain the hydrochloride salt of the compound of formula I (11.4 g, yield 96.1%, HPLC purity 99.2%).

### Example 5

Copper(I) iodide (0.29 g, 1.5 mmol), 2,2,2-trifluoro-1-phenyl-1-(5-phenyl-1H-pyrrol-2-yl)ethan-1-ol (L7) (0.48 g, 1.5 mmol), tert-butyl 6-cyano-2-(2-(4-ethyl-3-iodophenyl)propan-2-yl)-1H-indole-3-carboxylate (10.29 g, 20.0 mmol), 4-morpholinopiperidine (3.75 g, 22.0 mmol), and potassium hydroxide (3.37 g, 60.0 mmol) were placed in a dry reaction flask under a nitrogen atmosphere. Dimethyl sulfoxide (70 mL) was added to the flask, and the reaction mixture was then heated to 100°C and stirred at this temperature for 4 hours. The reaction mixture was cooled to room temperature, and isopropyl acetate (280 mL) and a 15% aqueous ammonium chloride solution (40 mL) were added. The resulting mixture was stirred at room temperature for 1 hour. The aqueous layer was separated, and a saturated aqueous sodium chloride solution (70 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (150 mL), and the internal temperature of the solution was raised to 35°C. A mixture of pyridine hydrochloride (2.54 g, 22.0 mmol) in ethanol (3.0 mL) and acetone (6.0 mL) was added dropwise over 30 minutes to crystallize the desired product. The resulting slurry was stirred at 0°C for 3 hours. The wet powder was collected by filtration through a Büchner funnel and washed with acetone (40 mL). The wet powder was dried under reduced pressure at 40°C for 5 hours to obtain the hydrochloride salt of the compound of formula I (10.8 g, yield 91.0%, HPLC purity 99.4%).

### Example 6

Copper(I) chloride (1.19 g, 12.0 mmol), 1,1,1-trifluoro-2-(1H-pyrrol-2-yl)propan-2-ol (L2) (2.15 g, 12.0 mmol), tert-butyl 6-cyano-2-(2-(4-ethyl-3-iodophenyl)propan-2-yl)-1H-indole-3-carboxylate (20.58 g, 40.0 mmol), 4-morpholinopiperidine (8.17 g, 48.0 mmol), and potassium carbonate (19.35 g, 140.0 mmol) were placed in a dry reaction flask under a nitrogen atmosphere. Dimethyl sulfoxide (120 mL) was added to the flask, and the reaction mixture was then heated to 105°C and stirred at this temperature for 3 hours. The reaction mixture was cooled to room temperature, and isopropyl acetate (360 mL) and a 15% aqueous ammonium chloride solution (60 mL) were added. The resulting mixture was stirred at room temperature for 1 hour. The aqueous layer was separated, and a saturated aqueous sodium chloride solution (120 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (250 mL), and the internal temperature of the solution was raised to 35°C. A mixture of pyridine hydrochloride (5.08 g, 44.0 mmol) in ethanol (6.0 mL) and acetone (12.0 mL) was added dropwise over 30 minutes to crystallize the desired product. The resulting slurry was stirred at 0°C for 3 hours. The wet powder was collected by filtration through a Büchner funnel and washed with acetone (50 mL). The wet powder was dried under reduced pressure at 40°C for 6 hours to obtain the hydrochloride salt of the compound of formula I (22.2 g, yield 93.6%, HPLC purity 99.2%).

### Example 7

Copper thiophene-2-carboxylate (0.76 g, 4.0 mmol), pyrrole alcohol ligand 1,1,1-trifluoro-2-(1H-pyrrol-2-yl)propan-2-ol (L2) (0.72 g, 4.0 mmol), tert-butyl 6-cyano-2-(2-(4-ethyl-3-iodophenyl)propan-2-yl)-1H-indole-3-carboxylate (20.58 g, 40.0 mmol), 4-morpholinopiperidine (8.17 g, 48.0 mmol), and triethylamine (14.17 g, 140.0 mmol) were placed in a dry reaction flask under a nitrogen atmosphere. N,N-Dimethylacetamide (120 mL) was added to the flask, and the reaction mixture was then heated to 115°C and stirred at this temperature for 6 hours. The reaction mixture was cooled to room temperature, and isopropyl acetate (360 mL) and a 15% aqueous ammonium chloride solution (60 mL) were added. The resulting mixture was stirred at room temperature for 1 hour. The aqueous layer was separated, and a saturated aqueous sodium chloride solution (120 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (250 mL), and the internal temperature of the solution was raised to 35°C. A mixture of pyridine hydrochloride (5.08 g, 44.0 mmol) in ethanol (6.0 mL) and acetone (12.0 mL) was added dropwise over 30 minutes to crystallize the desired product. The resulting slurry was stirred at 0°C for 3 hours. The wet powder was collected by filtration through a Büchner funnel and washed with acetone (50 mL). The wet powder was dried under reduced pressure at 40°C for 6 hours to obtain the hydrochloride salt of the compound of formula I (21.5 g, yield 90.6%, HPLC purity 98.7%).

### Example 8

Copper(I) oxide (1.72 g, 12.0 mmol), pyrrole alcohol ligand 1,1,1-trifluoro-2-(1H-pyrrol-2-yl)propan-2-ol (L2) (2.15 g, 12.0 mmol), tert-butyl 6-cyano-2-(2-(4-ethyl-3-iodophenyl)propan-2-yl)-1H-indole-3-carboxylate (20.58 g, 40.0 mmol), 4-morpholinopiperidine (8.17 g, 48.0 mmol), and triethylamine (14.17 g, 140.0 mmol) were placed in a dry reaction flask under a nitrogen atmosphere. Dimethyl sulfoxide (120 mL) was added to the flask, and the reaction mixture was then heated to 105°C and stirred at this temperature for 3 hours. The reaction mixture was cooled to room temperature, and isopropyl acetate (360 mL) and a 15% aqueous ammonium chloride solution (60 mL) were added. The resulting mixture was stirred at room temperature for 1 hour. The aqueous layer was separated, and a saturated aqueous sodium chloride solution (120 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (250 mL), and the internal temperature of the solution was raised to 35°C. A mixture of pyridine hydrochloride (5.08 g, 44.0 mmol) in ethanol (6.0 mL) and acetone (12.0 mL) was added dropwise over 30 minutes to crystallize the desired product. The resulting slurry was stirred at 0°C for 3 hours. The wet powder was collected by filtration through a Büchner funnel and washed with acetone (50 mL). The wet powder was dried under reduced pressure at 40°C for 6 hours to obtain the hydrochloride salt of the compound of formula I (22.8 g, yield 96.1%, HPLC purity 99.1%).

### Example 9

Copper(I) iodide (0.60 g, 6.0 mmol), pyrrole alcohol ligand 2,2,2-trifluoro-1-(1H-pyrrol-2-yl)ethan-1-ol (L1) (0.99 g, 6.0 mmol), tert-butyl 6-cyano-2-(2-(4-ethyl-3-iodophenyl)propan-2-yl)-1H-indole-3-carboxylate (20.58 g, 40.0 mmol), 4-morpholinopiperidine (8.17 g, 48.0 mmol), and potassium carbonate (16.59 g, 120.0 mmol) were placed in a dry reaction flask under a nitrogen atmosphere. Sulfolane (120 mL) was added to the flask, and the reaction mixture was then heated to 105°C and stirred at this temperature for 3 hours. The reaction mixture was cooled to room temperature, and isopropyl acetate (360 mL) and a 15% aqueous ammonium chloride solution (60 mL) were added. The resulting mixture was stirred at room temperature for 1 hour. The aqueous layer was separated, and a saturated aqueous sodium chloride solution (120 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (250 mL), and the internal temperature of the solution was raised to 35°C. A mixture of pyridine hydrochloride (5.08 g, 44.0 mmol) in ethanol (6.0 mL) and acetone (12.0 mL) was added dropwise over 30 minutes to crystallize the desired product. The resulting slurry was stirred at 0°C for 3 hours. The wet powder was collected by filtration through a Büchner funnel and washed with acetone (50 mL). The wet powder was dried under reduced pressure at 40°C for 6 hours to obtain the hydrochloride salt of the compound of formula I (23.1 g, yield 97.4%, HPLC purity 98.9%).

### Example 10

Copper(I) iodide (0.60 g, 6.0 mmol), pyrrole alcohol ligand 2,2,2-trifluoro-1-(1H-pyrrol-2-yl)ethan-1-ol (L1) (0.99 g, 6.0 mmol), tert-butyl 6-cyano-2-(2-(4-ethyl-3-(((trifluoromethyl)sulfonyl)oxy)phenyl)propan-2-yl)-1H-indole-3-carboxylate (21.46 g, 40.0 mmol), 4-morpholinopiperidine (8.17 g, 48.0 mmol), and potassium carbonate (16.59 g, 120.0 mmol) were placed in a dry reaction flask under a nitrogen atmosphere. Sulfolane (120 mL) was added to the flask, and the reaction mixture was then heated to 105°C and stirred at this temperature for 3 hours. The reaction mixture was cooled to room temperature, and isopropyl acetate (360 mL) and a 15% aqueous ammonium chloride solution (60 mL) were added. The resulting mixture was stirred at room temperature for 1 hour. The aqueous layer was separated, and a saturated aqueous sodium chloride solution (120 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (250 mL), and the internal temperature of the solution was raised to 35°C. A mixture of pyridine hydrochloride (5.08 g, 44.0 mmol) in ethanol (6.0 mL) and acetone (12.0 mL) was added dropwise over 30 minutes to crystallize the desired product. The resulting slurry was stirred at 0°C for 3 hours. The wet powder was collected by filtration through a Büchner funnel and washed with acetone (50 mL). The wet powder was dried under reduced pressure at 40°C for 6 hours to obtain the hydrochloride salt of the compound of formula I (22.7 g, yield 95.7%, HPLC purity 98.3%).

### Example 11

Copper(I) iodide (0.60 g, 6.0 mmol), pyrrole alcohol ligand 1,1,1-trifluoro-2-(1H-pyrrol-2-yl)propan-2-ol (L2) (1.07 g, 6.0 mmol), tert-butyl 2-(2-(3-bromo-4-ethylphenyl)propan-2-yl)-6-cyano-1H-indole-3-carboxylate (18.70 g, 40.0 mmol), 4-morpholinopiperidine (8.17 g, 48.0 mmol), and potassium phosphate (33.97 g, 160.0 mmol) were placed in a dry reaction flask under a nitrogen atmosphere. Dimethyl sulfoxide (120 mL) was added to the flask, and the reaction mixture was then heated to 120°C and stirred at this temperature for 6 hours. The reaction mixture was cooled to room temperature, and isopropyl acetate (360 mL) and a 15% aqueous ammonium chloride solution (60 mL) were added. The resulting mixture was stirred at room temperature for 1 hour. The aqueous layer was separated, and a saturated aqueous sodium chloride solution (120 mL) was added to the organic layer. The mixture was stirred at room temperature for 0.5 hours. The aqueous layer was separated, and the obtained organic layer was concentrated under reduced pressure at an external temperature of 50°C. The residue was dissolved in acetone (250 mL), and the internal temperature of the solution was raised to 35°C. A mixture of pyridine hydrochloride (5.08 g, 44.0 mmol) in ethanol (6.0 mL) and acetone (12.0 mL) was added dropwise over 30 minutes to crystallize the desired product. The resulting slurry was stirred at 0°C for 3 hours. The wet powder was collected by filtration through a Büchner funnel and washed with acetone (50 mL). The wet powder was dried under reduced pressure at 40°C for 6 hours to obtain the hydrochloride salt of the compound of formula I (20.3 g, yield 85.6%, HPLC purity 98.8%).

### Example 12

Copper(I) chloride (1.19 g, 12.0 mmol), pyrrole alcohol ligand 1,1,1-trifluoro-2-(1H-pyrrol-2-yl)propan-2-ol (L2) (2.15 g, 12.0 mmol), tert-butyl 6-cyano-2-(2-(4-ethyl-3-iodophenyl)propan-2-yl)-1H-indole-3-carboxylate (20.58 g, 40.0 mmol), 4-morpholinopiperidine (8.17 g, 48.0 mmol), and potassium phosphate (33.97 g, 160.0 mmol) were placed in a dry reaction flask under a nitrogen atmosphere. Dimethyl sulfoxide (120 mL) was added to the flask, and the reaction mixture was then heated to 110°C and stirred at this temperature for 3 hours. The reaction mixture was cooled to room temperature, and 2M aqueous NaOH solution (6.0 mL) was added to the reaction system, followed by stirring at room temperature for 1 hour. The mixture was then filtered through a Büchner funnel, and the filter cake was washed with dimethyl sulfoxide (20 mL). While stirring at room temperature, 1M aqueous HCl solution (360 mL) was slowly added dropwise to the filtrate, resulting in precipitation of a yellow solid. The mixture was stirred at room temperature for 3 hours, then filtered. The filter cake was washed sequentially with water (100 mL) and acetone (100 mL). The wet powder was dried under reduced pressure at 40°C for 6 hours to obtain the hydrochloride salt of the compound of formula I (22.6 g, yield 95.2%, HPLC purity 98.2%).

### Comparison Example

Subhajit Bhunia et al., in the publication "Selected Copper-Based Reactions for C-N, C-O, C-S and C-C Bond Formation" (Angew. Chem. Int. Ed. 2017, 56, 16136), disclose reactions using copper catalyst reaction systems. Eight ligands (1a-1h) were investigated for use in a copper-catalyzed carbon-nitrogen bond coupling system to prepare an alectinib intermediate, the compound of formula I, or a salt thereof. The reaction results are shown in Table 1. The complexes formed between various ligands and copper(I) iodide exhibited poor catalytic activity, and the aryl iodide (II) and 4-morpholinopiperidine (III) scarcely reacted.

**Table 1:**

| | | | |
|---|---|---|---|
| 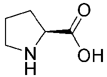 1a 20% yield | 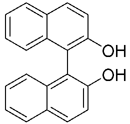 1b < 5% yield | 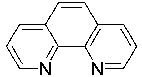 1c NR | 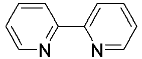 1d NR |
| 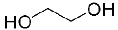 1e NR | 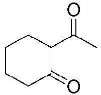 1f < 5% yield | 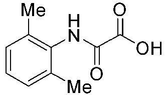 1g < 5% yield | 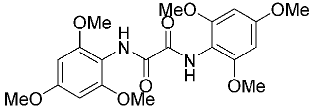 1h < 5% yield |

### Comparison Example 1

Copper(I) iodide (0.74 g, 3.9 mmol) and L-proline (1a) (0.90 g, 7.8 mmol) were placed in a dry reaction flask under a nitrogen atmosphere. Dimethyl sulfoxide (60 mL) was added to the flask, and the mixture was stirred at 25°C for 1 hour. Subsequently, tert-butyl 6-cyano-2-(2-(4-ethyl-3-iodophenyl)propan-2-yl)-1H-indole-3-carboxylate (10.0 g, 19.4 mmol), 4-morpholinopiperidine (6.61 g, 38.8 mmol), and cesium carbonate (19.0 g, 58.2 mmol) were added to the reaction flask under a nitrogen atmosphere. The reaction mixture was heated to 110°C and stirred at this temperature for 24 hours. The reaction mixture was cooled to room temperature, and isopropyl acetate (240 mL) and a 15% aqueous ammonium chloride solution (60 mL) were added. The resulting mixture was stirred at room temperature for 1 hour. The aqueous layer was separated, and a saturated aqueous sodium chloride solution (60 mL) was added to the organic layer. The mixture was stirred at room temperature for 1 hour. The aqueous layer was separated, and the obtained organic layer was concentrated under reduced pressure at an external temperature of 50°C. The concentrate was purified by column chromatography (dichloromethane/methanol = 30/1) to obtain the compound of formula I (2.16 g, yield 20.0%, HPLC purity 98.2%).

### Comparison Example 2

Copper(I) iodide (0.74 g, 3.9 mmol) and 2-acetylcyclohexan-1-one (1f) (1.09 g, 7.8 mmol) were placed in a dry reaction flask under a nitrogen atmosphere. Dimethyl sulfoxide (30 mL) was added to the flask, and the mixture was stirred at 25°C for 1 hour. Subsequently, tert-butyl 6-cyano-2-(2-(4-ethyl-3-iodophenyl)propan-2-yl)-1H-indole-3-carboxylate (10.0 g, 19.4 mmol), 4-morpholinopiperidine (6.61 g, 38.8 mmol), and cesium carbonate (12.6 g, 38.8 mmol) were added to the reaction flask under a nitrogen atmosphere. The reaction mixture was heated to 100°C and stirred at this temperature for 24 hours. The reaction mixture was cooled to room temperature, and isopropyl acetate (120 mL) and a 15% aqueous ammonium chloride solution (30 mL) were added. The resulting mixture was stirred at room temperature for 1 hour. The aqueous layer was separated, and a saturated aqueous sodium chloride solution (30 mL) was added to the organic layer. The mixture was stirred at room temperature for 1 hour. The aqueous layer was separated, and the obtained organic layer was concentrated under reduced pressure at an external temperature of 50°C. The concentrate was purified by column chromatography (dichloromethane/methanol = 30/1) to obtain the compound of formula I (0.46 g, yield 4.3%, HPLC purity 98.8%).

### Comparison Example 3

Copper(I) iodide (0.74 g, 3.9 mmol) and N₁,N₂-bis(2,4,6-trimethoxyphenyl)oxalamide (1h) (1.64 g, 3.9 mmol) were placed in a dry reaction flask under a nitrogen atmosphere. Dimethyl sulfoxide (50 mL) was added to the flask, and the mixture was stirred at 25°C for 1 hour. Subsequently, tert-butyl 6-cyano-2-(2-(4-ethyl-3-iodophenyl)propan-2-yl)-1H-indole-3-carboxylate (10.0 g, 19.4 mmol), 4-morpholinopiperidine (6.61 g, 38.8 mmol), and potassium phosphate (8.2 g, 38.8 mmol) were added to the reaction flask under a nitrogen atmosphere. The reaction mixture was heated to 110°C and stirred at this temperature for 24 hours. The reaction mixture was cooled to room temperature, and isopropyl acetate (200 mL) and a 15% aqueous ammonium chloride solution (50 mL) were added. The resulting mixture was stirred at room temperature for 1 hour. The aqueous layer was separated, and a saturated aqueous sodium chloride solution (50 mL) was added to the organic layer. The mixture was stirred at room temperature for 1 hour. The aqueous layer was separated, and the obtained organic layer was concentrated under reduced pressure at an external temperature of 50°C. The concentrate was purified by column chromatography (dichloromethane/methanol = 30/1) to obtain the compound of formula I (0.25 g, yield 2.3%, HPLC purity 98.5%).

It will be obvious to those skilled in the art that changes and modifications may be made, and therefore, the aim in the appended claims is to cover all such changes and modifications.

## Claims

1. A method for preparing an intermediate of an anti-cancer drug, comprising:
reacting a compound of formula II with a compound of formula III, 4-morpholinopiperidine, in the presence of a cuprous salt as a catalyst and a pyrrole alcohol compound as a ligand, to obtain a compound of formula I or a salt thereof:

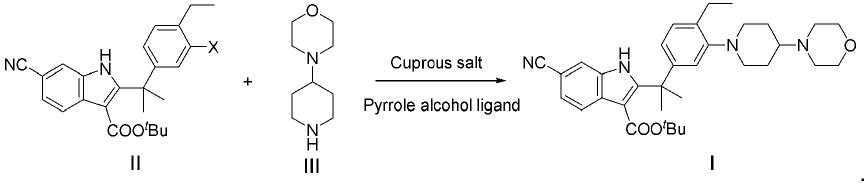

2. The method of claim 1, wherein the pyrrole alcohol compound has a formula as follows: 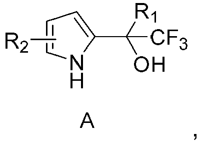
R₁ is selected from the group consisting of H, methyl, ethyl, straight-chain or branched C₃-C₅ alkyl, benzyl, phenyl, naphthyl, and substituted phenyl; wherein the substituted phenyl is substituted at an ortho, meta, or para position with a substituent selected from the group consisting of methyl, methoxy, and isopropyl; and
R₂ is selected from the group consisting of H, methyl, ethyl, straight-chain or branched C₃-C₅ alkyl, phenyl, benzyl, methoxy, and ethoxy, and R₂ is located at any substitutable position on a pyrrole ring.

3. The method of claim 2, wherein R₁ is selected from the group consisting of H, methyl, ethyl, straight-chain or branched C₃-C₅ alkyl, and phenyl; R₂ is selected from the group consisting of H, methyl, ethyl, and phenyl, and R₂ is located at any position selected from the 3-position, 4-position, or 5-position of the pyrrole ring.

4. The method of claim 3, wherein the pyrrole alcohol compound comprises the following formulas:

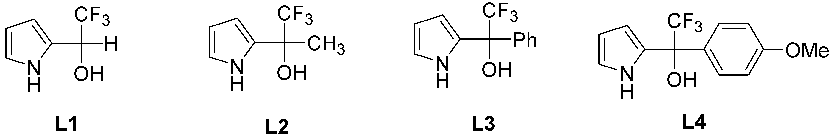

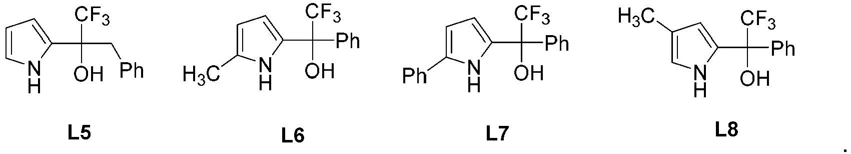

5. The method of claim 1, wherein the cuprous salt is selected from the group consisting of cuprous iodide, cuprous bromide, cuprous chloride, cuprous oxide, and copper thiophene-2-carboxylate.

6. The method of claim 1, wherein a molar ratio of the cuprous salt to the compound of formula II is from 1: 1 to 1: 20.

7. The method of claim 6, wherein the molar ratio of the cuprous salt to the compound of formula II is from 1: 1 to 1: 15.

8. The method of claim 1, wherein reacting the compound of formula II with the compound of formula III is carried out at a temperature ranging from 80°C to 130°C for a reaction time ranging from 1 hour to 15 hours.

9. The method of claim 8, wherein reacting the compound of formula II with the compound of formula III is carried out at a temperature ranging from 90°C to 125°C for a reaction time ranging from 1 hour to 12 hours.

10. The method of claim 1, wherein reacting the compound of formula II with the compound of formula III further involves a base selected from the group consisting of triethylamine, diisopropylethylamine, 1,8-diazabicyclo[5.4.0]undec-7-ene, potassium phosphate, potassium carbonate, cesium carbonate, and potassium hydroxide, and a molar ratio of the base to the compound of formula II is from 1: 1 to 6: 1.
